# NOUVEAU FASCICULE DE BREVET EUROPEEN

(11) **EP 1 010 413 B2**
(45) Date de publication et mention de la décision concernant l'opposition: **29.12.2010**
(45) Mention de la délivrance du brevet: 20.11.2002
(21) Numéro de dépôt: 99402836.3
(22) Date de dépôt: 16.11.1999
(51) Int. Cl.: A61K 8/06

(54) **Nanoémulsion à base d'esters gras de sucre ou d'éthers gras de sucre et ses utilisations dans les domaines cosmétique, dermatologique et/ou ophtalmologique**
Nanoemulsion, bestehend aus Fettsauereester von Zuckern oder Fettalkoholether von Zuckern und ihre Verwendung in Kosmetika, Dermatologie und/oder Ophthalmologie
Nanoemulsion based on fatty acid esters of sugar or fatty ethers of sugar and uses thereof in the cosmetical, dermatological and/or ophtalmological fields

(30) Priorité: 14.12.1998 FR 9815765
(43) Date de publication de la demande: 21.06.2000
(73) Titulaire: L'Oréal, 75008 Paris (FR)
(72) Inventeur: Simonnet, Jean-Thierry, 75011 Paris (FR); Sonneville, Odile, 75014 Paris (FR); Legret, Sylvie, 92320 Chatillon (FR)
(74) Mandataire: Dossmann, Gérard

(56) Documents cités:
- EP-A- 0 728 460
- EP-A- 0 956 851

## Description

La présente invention concerne une nanoémulsion à base d'un tensioactif solide à une température égale à 45°C, choisi parmi les esters d'acide gras et de sucre et les éthers d'alcool gras et de sucre, et d'au moins une huile ayant un poids moléculaire supérieur à 400, le rapport en poids de la quantité de phase huileuse sur la quantité de tensioactif allant de 2 à 10.

L'invention se rapporte aussi au procédé de préparation de la dite nanoémulsion et à ses utilisations dans les domaines cosmétique, dermatologique et/ou ophtalmologique. Cette nanoémulsion est stable au stockage et peut contenir des quantités importantes d'huile tout en conservant une bonne transparence et en ayant de bonnes propriétés cosmétiques.

Les nanoémulsions sont des émulsions huile-dans-eau dont les globules d'huile ont une granulométrie très fine, c'est-à-dire une taille moyenne en nombre, inférieure à 100 nm. Elles sont généralement fabriquées par fragmentation mécanique d'une phase huileuse dans une phase aqueuse en présence de tensioactif. Dans le cas des nanoémulsions, la très petite taille des globules huileux est obtenue notamment grâce à au moins un passage dans un homogénéiseur haute-pression. La petite taille des globules leur confère des propriétés intéressantes sur le plan cosmétique, qui les distinguent des émulsions classiques : elles sont transparentes et présentent une texture originale. Elles peuvent également véhiculer les actifs de façon plus efficace.

On connaît dans l'état de la technique des microémulsions transparentes. Les microémulsions ne sont pas à proprement parler des émulsions, contrairement aux nanoémulsions ; ce sont des solutions transparentes de micelles gonflées par de l'huile qui est en général à chaîne très courte (ex : hexane, décane) et qui est solubilisée grâce à la présence conjointe d'une quantité importante de tensioactifs et de co-tensioactifs formant les micelles. La taille des micelles gonflées est très petite en raison de la faible quantité d'huile qu'elles peuvent solubiliser. Cette très petite taille des micelles est la cause de leur transparence comme pour les nanoémulsions. Cependant, contrairement aux nanoémulsions, les microémulsions se forment spontanément par mélange des constituants sans apport d'énergie mécanique autre qu'une simple agitation magnétique. Les inconvénients majeurs des microémulsions sont liés à leur forte proportion en tensioactifs, conduisant à des intolérances et entraînant un toucher collant lors de l'application sur la peau. Par ailleurs, leur domaine de formulation est en général très étroit et leur stabilité en température très limitée.

En outre, on connaît dans l'état de la technique des nanoémulsions comprenant une phase lipidique amphiphile constituée de phospholipides, d'eau et d'huile. Ces émulsions présentent l'inconvénient d'être instables au stockage aux températures traditionnelles de conservation, à savoir entre 0 et 45°C. Elles conduisent à des compositions jaunes et produisent des odeurs de rance qui se développent après quelques jours de conservation.

On connaît aussi des nanoémulsions stabilisées par un enrobage cristal liquide lamellaire obtenu par l'association d'un tensioactif hydrophile et d'un tensioactif lipophile. Toutefois, ces associations sont délicates à déterminer. De plus, les nanoémulsions obtenues présentent un toucher cireux et filmogène peu agréable pour l'utilisateur.

Par ailleurs, le document EP-A-728 460 décrit des nanoémulsions à base de lipides amphiphiles non-ioniques fluides. Toutefois, ces nanoémulsions présentent l'inconvénient d'avoir un effet collant lors de l'application sur la peau.

Il subsiste donc le besoin de nanoémulsions n'ayant ni les inconvénients de celles de l'art antérieur ni les inconvénients des microémulsions.

La demanderesse a maintenant découvert, de façon inattendue, que l'utilisation d'un tensioactif solide.à une température égale à 45°C, choisi parmi les esters ou éthers de sucre, et d'au moins une huile ayant un poids moléculaire supérieur à 400 (c'est-à-dire 400 grammes par mole) permettait d'obtenir de nouvelles nanoémulsions présentant tous les avantages des nanoémulsions connues sans leurs inconvénients.

La présente invention a pour objet une nanoémulsion comportant une phase huileuse dispersée dans une phase aqueuse et ayant des globules d'huile dont la taille moyenne en nombre est inférieure à 100 nm, caractérisée en ce qu'elle contient un tensioactif solide à une température égale à 45°C, choisi parmi les esters d'acide gras et de sucre et les éthers d'alcool gras et de sucre, et au moins une huile ayant un poids moléculaire supérieur à 400, et en ce que le rapport en poids de la quantité de phase huileuse sur la quantité de tensioactif va de 2 à 10.

Les nanoémulsions selon l'invention ont généralement un aspect transparent à bleuté. Leur transparence se mesure par un coefficient de transmittance à 600 nm allant de 10 à 90 % ou bien par une turbidité allant de 60 à 600 NTU et de préférence de 70 à 300 NTU, turbidité mesurée au turbidimètre portatif HACH - Modèle 2100 P.

Les globules d'huile des nanoémulsions de l'invention ont une taille moyenne en nombre, inférieure à 100 nm et de préférence allant de 20 à 75 nm et plus préférentiellement de 40 à 60 nm. La diminution de la taille des globules permet de favoriser la pénétration des actifs dans les couches superficielles de la peau (effet véhicule).

Le tensioactif solide à une température égale à 45°C, utilisable dans la nanoémulsion de l'invention est exclusivement choisi parmi les esters d'acide gras et de sucre, les éthers d'alcool gras et de sucre, et leurs mélanges, ce qui signifie que la nanoémulsion de l'invention est exempte de tout tensioactif autre que les esters gras ou éthers gras de sucre.

Les esters d'acide gras et de sucre, utilisables comme tensioactifs dans la nanoémulsion selon l'invention sont solides à une température égale à 45°C et peuvent être choisis notamment dans le groupe comprenant les esters ou les mélanges d'esters d'acide gras en C₈-C₂₂ et de sucrose, de maltose, de glucose ou de fructose, et les esters ou les mélanges d'esters d'acide gras en C₁₄-C₂₂ et de méthylglucose.

Les acides gras en C₈-C₂₂ ou en C₁₄-C₂₂ formant le motif gras des esters utilisables dans la nanoémulsion de l'invention comportent une chaîne alkyle linéaire saturée ou non saturée, comportant respectivement de 8 à 22 ou de 14 à 22 atomes de carbone. Le motif gras des esters peut être notamment choisi parmi les stéarates, béhénates, arachidonates, palmitates, myristates, laurates, caprates et leurs mélanges. On utilise de préférence des stéarates.

On peut citer, à titre d'exemples d'esters ou de mélanges d'esters d'acide gras et de sucrose, de maltose, de glucose ou de fructose, le monostéarate de sucrose, le distéarate de sucrose, le tristéarate de sucrose et leurs mélanges, tels que les produits commercialisés par la société Croda sous la dénomination Crodesta F50, F70, F110, F160 ayant respectivement un HLB (Hydrophilic Lipophilic Balance) de 5, 7, 11 et 16 ; et à titre d'exemples d'esters ou de mélanges d'esters d'acide gras et de méthylglucose, le distéarate de méthyl glucose et de polyglycérol-3, vendu par la société Goldschmidt sous la dénomination de Tego-care 450. On peut citer aussi les monoesters de glucose ou de maltose tels que l'O-hexadécanoyle-6-D-glucoside de méthyle et l'O-hexadécanoyle-6-D-maltoside de méthyle.

Les éthers d'alcool gras et de sucre, utilisables comme tensioactifs dans la nanoémulsion selon l'invention sont solides à une température égale à 45°C et peuvent être choisis notamment dans le groupe comprenant les éthers ou mélanges d'éthers d'alcool gras en C₈-C₂₂ et de glucose, de maltose, de sucrose ou de fructose et les éthers ou mélanges d'éthers d'alcool gras en C₁₄-C₂₂ et de méthylglucose. Ce sont notamment des alkylpolyglucosides.

Les alcools gras en C₈-C₂₂ ou en C₁₄-C₂₂ formant le motif gras des éthers utilisables dans la nanoémulsion de l'invention comportent une chaîne alkyle linéaire saturée ou non saturée, comportant respectivement de 8 à 22 ou de 14 à 22 atomes de carbone. Le motif gras des éthers peut être notamment choisi parmi les motifs décyl, cétyle, béhényle, arachidyle, stéaryle, palmityle; myristyle, lauryle, capryle, hexadécanoyle, et leurs mélanges tels que cétéaryle.

A titre d'exemples d'éthers d'alcool gras et de sucre, on peut citer les alkylpolyglucosides tels que le décyl glucoside et le lauryl glucoside commercialisés par exemple par la société Henkel sous les dénominations respectives Plantaren 2000 et Plantaren 1200, le cétostéaryl glucoside éventuellement en mélange avec l'alcool cétostéarylique, commercialisé par exemple sous la dénomination Montanov 68 par la société Seppic, sous la dénomination Tego-care CG90 par la société Goldschmidt et sous la dénomination Emulgade KE3302 par la société Henkel, ainsi que l'arachidyl glucoside, par exemple sous la forme du mélange d'alcools arachidique et béhénique et d'arachidyl glucoside commercialisé sous la dénomination Montanov 202 par la société Seppic.

Selon un mode particulier de réalisation de l'invention, on utilise plus particulièrement comme tensioactif le monostéarate de sucrose, le distéarate de sucrose, le tristéarate de sucrose et leurs mélanges, le distéarate de méthyl glucose et de polyglycérol-3 et les alkylpolyglucosides.

Selon son caractère plus hydrophile ou plus lipophile, le tensioactif peut être introduit dans la phase aqueuse ou dans la phase huileuse de la nanoémulsion.

La quantité de tensioactif dans la nanoémulsion de l'invention peut aller par exemple de 0,2 à 15 % en poids et de préférence de 1 à 8 % en poids par rapport au poids total de la nanoémulsion.

Le rapport en poids de la quantité de la phase huileuse sur la quantité de tensioactif va de 2 à 10 et, de préférence, de 3 à 6. On entend ici par "quantité de phase huileuse" la quantité totale des constituants de cette phase sans inclure la quantité de tensioactif.

La nanoémulsion selon l'invention contient au moins une huile de poids moléculaire supérieur à 400. Les huiles de poids moléculaire supérieur à 400 peuvent être choisies parmi les huiles d'origine animale ou végétale, les huiles minérales, les huiles de synthèse et les huiles de silicone, et leurs mélanges. Comme huiles de ce type, on peut citer par exemple le palmitate d'isocétyle, le stéarate d'isocétyle, l'huile d'avocat, l'huile de jojoba.

En outre, la phase huileuse peut éventuellement contenir d'autres huiles et notamment des huiles ayant un poids moléculaire inférieur à 400. Ces huiles sont choisies également parmi les huiles d'origine animale ou végétale, les huiles minérales, les huiles de synthèse et les huiles de silicone. On peut citer par exemple comme huiles de poids moléculaire inférieur à 400, l'isododécane, l'isohexadécane, les huiles de silicone volatiles, le myristate d'isopropyle, le palmitate d'isopropyle, l'isoparaffine C11-C13.

La phase huileuse peut contenir également des corps gras autres que les huiles indiquées ci-dessus, tels que les alcools gras comme les alcools stéarylique, cétylique, béhénique, les acides gras comme les acides stéarique, palmitique et béhénique, les huiles fluorées, les cires, les gommes et leurs mélanges.

Les nanoémulsions conformes à l'invention comportent une quantité de phase huileuse allant de préférence de 2 à 40 % et mieux de 5 à 30 % en poids par rapport au poids total de la nanoémulsion, la proportion d'huile(s) ayant un poids moléculaire supérieur à 400 représentant de préférence au moins 40 % en poids de la phase huileuse.

Selon une forme particulière de réalisation de l'invention, la nanoémulsion de l'invention contient en outre un ou plusieurs lipides amphiphiles ioniques.

Les lipides amphiphiles ioniques pouvant être utilisés dans les nanoémulsions de l'invention sont choisis de préférence dans le groupe formé par les lipides amphiphiles anioniques, les lipides amphiphiles cationiques et les dérivés alkylsulfoniques.

Les lipides amphiphiles anioniques peuvent être plus particulièrement choisis dans le groupe formé par :
- les sels alcalins du dicetyl- et du dimyristylphosphate ;
- les sels alcalins du cholestérol sulfate ;
- les sels alcalins du cholestérol phosphate ;
- les lipoaminoacides et leurs sels tels que les acylglutamates mono- et disodiques comme le sel disodique de l'acide N-stéaroyl L-glutamique commercialisé sous la dénomination Acylglutamate HS21 par la société AJINOMOTO ;
- les sels de sodium de l'acide phosphatidique ;
- les phospholipides.

Les dérivés alkylsulfoniques peuvent être plus particulièrement choisis parmi les dérivés alkylsulfoniques de formule (I) : 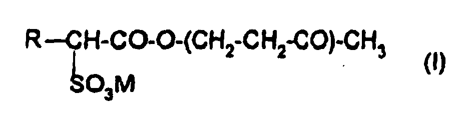 dans laquelle R représente un radical alkyle comportant de 16 à 22 atomes de carbone, en particulier les radicaux C₁₆H₃₃ et C₁₈H₃₇ pris en mélange ou séparément, et M est un métal alcalin tel que le sodium.

Les lipides amphiphiles cationiques peuvent être plus particulièrement choisis dans le groupe formé par les sels d'ammonium quaternaire, les amines grasses et leurs sels.

Les sels d'ammonium quaternaires sont par exemple :
- ceux qui présentent la formule générale (II) suivante : 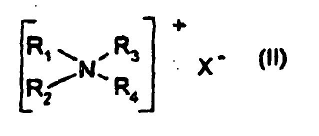 dans laquelle les radicaux R₁ à R₄, qui peuvent être identiques ou différents, représentent un radical aliphatique, linéaire ou ramifié, comportant de 1 à 30 atomes de carbone, ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, polyoxyalkylène(C₂-C₆), alkylamide, alkyl(C₁₂-C₂₂)amido alkyle(C₂-C₆), alkyl(C₁₂-C₂₂)acétate, hydroxyalkyle, comportant environ de 1 à 30 atomes de carbone ; X est un anion choisi dans le groupe des halogénures, phosphates, acétates, lactates, alkyl(C₂-C₆)sulfates, alkyl-ou-alkylarylsulfonates. Comme sels d'ammonium quaternaire de formule (II), on préfère, d'une part, les chlorures de tétraalkylammonium comme par exemple les chlorures de dialkyldiméthylammonium ou d'alkyltriméthylammonium, dans lesquels le radical alkyle comporte environ de 12 à 22 atomes de carbone, en particulier les chlorures de béhényltriméthylammonium, de distéaryldiméthyl-ammonium, de cétyltriméthyl-ammonium, de benzyl diméthyl stéaryl-ammonium ou encore, d'autre part, le chlorure de stéaramidopropyldiméthyl (myristyl acetate) ammonium vendu sous la dénomination «CERAPHYL 70» par la société VAN DYK.
- les sels d'ammonium quaternaire de l'imidazolinium, comme par exemple ceux de formule (III) suivante : 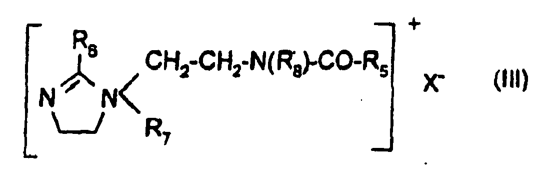 dans laquelle R₅ représente un radical alcényle ou alkyle comportant de 8 à 30 atomes de carbone, par exemple dérivé des acides gras du suif ; R₆ représente un atome d'hydrogène, un radical alkyle comportant de 1 à 4 atomes de carbone ou un radical alcényle ou alkyle comportant de 8 à 30 atomes de carbone ; R₇ représente un radical alkyle comportant de 1 à 4 atomes de carbone ; R₈ représente un atome d'hydrogène, un radical alkyle comportant de 1 à 4 atomes de carbone ; X est un anion choisi dans le groupe des halogénures, phosphates, acétates, lactates, alkylsulfates, alkyl-ou-alkylarylsulfonates. De préférence, R₅ et R₆ désignent un mélange de radicaux alcényle ou alkyle comportant de 12 à 21 atomes de carbone, par exemple dérivés des acides gras du suif, R₇ désigne un radical méthyle, R₈ désigne l'hydrogène. Un tel produit est par exemple vendu sous la dénomination «REWOQUAT W 75» par la société REWO.
- les sels de diammonium quaternaire de formule (IV) : 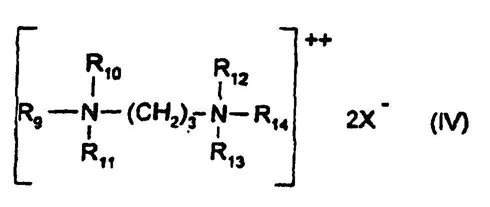 dans laquelle R₉ désigne un radical aliphatique comportant environ de 16 à 30 atomes de carbone ; R₁₀, R₁₁, R₁₂, R₁₃, et R₁₄ sont choisis parmi l'hydrogène ou un radical alkyle comportant de 1 à 4 atomes de carbone ;et X est un anion choisi dans le groupé des halogénures, acétates, phosphates, nitrates et méthylsulfates.

De tels sels de diammonium quaternaire comprennent notamment le dichlorure de propanesuif diammonium.

Selon un mode préféré de réalisation de l'invention, on utilise comme lipide amphiphile ionique un lipoaminoacide.

Les lipides ioniques amphiphiles peuvent être introduits dans l'une ou l'autre phase de la nanoémulsion. Lorsqu'ils sont présents dans la nanoémulsion de l'invention, ils peuvent être utilisés dans des concentrations allant de préférence de 0,01 à 5 % en poids et plus particulièrement de 0,25 à 1 % en poids par rapport au poids total de la nanoémulsion.

Les émulsions conformes à la présente invention peuvent contenir des additifs pour améliorer la transparence de la formulation.

Ces additifs sont choisis de préférence dans le groupe formé par :
- les alcools inférieurs comportant de 1 à 8 atomes de carbone et plus particulièrement de 2 à 6 atomes de carbone, tels que l'éthanol ;
- les glycols tels que la glycérine, le propylène glycol, le 1,3- butylène glycol, le dipropylène glycol, le pentylène glycol, l'isoprène glycol et les polyéthylèneglycols comportant de 4 à 16 et de préférence de 8 à 12 unités d'oxyde d'éthylène ;
- les sucres tels que le glucose, le fructose, le maltose, le lactose, le sucrose.

Ces additifs peuvent être utilisés en mélange. Lorsqu'ils sont présents dans la nanoémulsion de l'invention, ils peuvent être utilisés à dès concentrations allant de préférence de 0,01 à 30 % en poids par rapport au poids total de la nanoémulsion, et mieux de 5 à 20 % en poids par rapport au poids total de la nanoémulsion. La quantité d'alcool(s) et/ou de sucre(s) va de préférence de 5 à 20 % en poids par rapport au poids total de la nanoémulsion et la quantité de glycol(s) va de préférence de 5 à 15 % en poids par rapport au poids total de la nanoémulsion.

En outre, l'utilisation des alcools tels que définis ci-dessus, à des concentrations supérieures ou égales à 15 % en poids permet d'obtenir des émulsions sans conservateur.

Les nanoémulsions définies ci-dessus peuvent constituer des compositions à usage topique et notamment cosmétiques ou dermatologiques. Elles peuvent aussi être utilisées comme supports ophtalmiques.

Un autre objet de l'invention consiste donc en une composition à usage topique, caractérisée en ce que qu'elle est constituée par une nanoémulsion telle que définie précédemment.

Une composition à usage topique contient un milieu physiologiquement acceptable, c'est-à-dire compatible avec la peau, les muqueuses, le cuir chevelu, les yeux et/ou les cheveux.

L'invention a aussi pour objet un support ophtalmique, caractérisé en ce qu'il est constitué d'une nanoémulsion telle que définie précédemment.

Les nanoémulsions de l'invention peuvent contenir des actifs hydrosolubles ou liposolubles ayant une activité cosmétique, dermatologique ou ophtalmique. Les actifs liposolubles sont dans les globules huileux de l'émulsion, tandis que les actifs hydrosolubles sont dans la phase aqueuse de l'émulsion. On peut citer, à titre d'exemples d'actifs, les vitamines telles que la vitamine E, et leurs dérivés et en particulier leurs esters, les provitamines telles que le panthénol, les humectants et les filtres solaires.

Comme actifs ophtalmiques, on peut citer par exemple les agents anti-glaucome, tels que le betaxolol ; les antibiotiques tels que l'acyclovir ; les antiallergiques ; les agents anti-inflammatoires tels que l'ibuprofène et ses sels, le diclofénac et ses sels, l'indométhacine ; les agents antiviraux.

Les nanoémulsions conformes à l'invention peuvent se présenter sous forme de lotion, de sérum, de crème, de lait ou d'eau de toilette et peuvent contenir des adjuvants habituellement utilisés dans les domaines cosmétique, dermatologique et ophtalmique, tels que par exemple les gélifiants, les conservateurs, les antioxydants et les parfums. Elles peuvent aussi se présenter sous forme de collyre, en particulier pour les applications ophtalmologiques.

Parmi les gélifiants utilisables, on peut citer les dérivés de cellulose, les dérivés d'algues, les gommes naturelles et les polymères synthétiques tels que les polymères et copolymères d'acides carboxyvinyliques comme ceux commercialisés sous la dénomination CARBOPOL par la société GOODRICH.

L'invention a aussi pour objet un procédé de préparation d'une nanoémulsion telle que définie ci-dessus, ce procédé consistant à mélanger la phase aqueuse et la phase huileuse sous agitation vive, à une température allant de 10 à 80°C, puis à effectuer une homogénéisation à une pression allant de préférence de 6.10⁷ Pa à 18.10⁷ Pa (homogénéisation haute pression). Le cisaillement va de préférence de 2.10⁶ s⁻¹ à 5.10⁸ s⁻¹ et mieux de 1.10⁸ s-¹ à 3.10⁸ s⁻¹ (s⁻¹ signifie seconde⁻¹).

La nanoémulsion de l'invention peut être par exemple utilisée pour le soin, le traitement, le maquillage de la peau, du visage et/ou du cuir chevelu.

L'invention a donc aussi pour objet l'utilisation cosmétique de la nanoémulsion telle que définie ci-dessus pour le soin, le traitement et/ou le maquillage de la peau, du visage et/ou du cuir chevelu.

En outre, la nanoémulsion de l'invention peut aussi être utilisée pour le soin et/ou le traitement des cheveux. Elle permet d'obtenir un dépôt d'huile sur les cheveux, ce qui rend ceux-ci plus brillants, plus résistants au coiffage, sans toutefois les alourdir. Elle permet aussi, en prétraitement d'améliorer les effets de la coloration ou de la permanente.

L'invention a donc aussi pour objet l'utilisation cosmétique de la nanoémulsion telle que définie ci-dessus pour le soin et/ou le traitement des cheveux.

La nanoémulsion selon l'invention permet notamment une bonne hydratation de la peau, des muqueuses et/ou du cuir chevelu, et est particulièrement adaptée au traitement de la peau sèche.

Un autre objet de l'invention est donc un procédé cosmétique de soin et/ou d'hydratation de la peau, des muqueuses et/ou du cuir chevelu, caractérisé en ce qu'on applique sur la peau, les muqueuses et/ou le cuir chevelu une nanoémulsion telle que définie ci-dessus.

L'invention porte également sur l'utilisation de la nanoémulsion selon l'invention pour la fabrication d'une composition dermatologique destinée au traitement de la peau sèche.

Enfin, l'invention porte aussi sur l'utilisation de la nanoémulsion selon l'invention pour la fabrication d'une composition ophtalmologique.

Les exemples qui suivent, permettront de mieux comprendre l'invention, sans toutefois présenter un caractère limitatif. Les quantités indiquées sont en % en poids.

### Exemple 1 : Fluide démaquillant

### Phase huileuse :

- Tego-Care 450 (Société GOLDSCHMIDT) 4,5 %
- Sel disodique de l'acide N-Stéaroyl L-glutamique (Acylglutamate HS21 de la société AJINOMOTO) 0,5 %
- Stéarate d'isocétyle (P. M. = 508) 10 %
- Myristate d'isopropyle (P.M. = 270) 5 %

### Phase aqueuse :

- Glycérine 5 %
- Dipropylène glycol 10 %
- Eau 65 %

On obtient une nanoémulsion transparente dont la taille des globules est de 49 nm et la turbidité de 218 NTU.

### Exemple 2 : Gel démaquillant

### Phase huileuse :

- Crodesta F50 (Société CRODA) 4,5 %
- Sel disodique de l'acide N-Stéaroyl L-glutamique (Acylglutamate HS21 de la société AJINOMOTO) 0,5 %
- Stéarate d'isocétyle (P.M. = 508) 20 %
- C11-C13 isoparaffine (P.M. = 170) 2,5 %
- Isohexadécane (P.M. = 226) 2,5 %

### Phase aqueuse :

- Glycérine 5 %
- Dipropylène glycol 10 %
- Eau 55 %

On obtient une nanoémulsion transparente gélifiée dont la taille des globules est de 45 nm et la turbidité de 260 NTU.

### Exemple 3 : Eau parfumée

### Phase huileuse :

- Crodesta F70 (Société CRODA) 4,5 %
- Sel disodique de l'acide N-Stéaroyl L-glutamique (Acylglutamate HS21) (Société AJINOMOTO) 0,5 %
- Huile de soja (P.M. de l'ordre de 900) 6 %
- Huile de silicone volatile (P.M. = 106) 2 %
- Parfum 3 %
- Acétate de vitamine E 0,5 %
- Ethanol 10 %

### Phase aqueuse:

- Glycérine 5 %
- Eau 68,5 %
On obtient une nanoémulsion transparente dont la taille des globules est de 39nm et la turbidité de 96 NTU.

### Exemple 4 : Fluide démaquillant

### Phase huileuse :

- Tego-Care CG90 (Société GOLDSCHMIDT) 4,5 %
- Sel disodique de l'acide N-Stéaroyl L-glutamique (Acylglutamate HS21 de la société AJINOMOTO) 0,5 %
- Stéarate d'isocétyle (P.M. = 508) 10 %
- Myristate d'isopropyle (P.M. = 270) 5 %

### Phase aqueuse :

- Glycérine 5 %
- Dipropylène glycol 10 %
- Eau 65 %

On obtient une nanoémulsion transparente dont la taille des globules est de 43 nm et la turbidité de 145 NTU.

### Exemple 5 : Gel de soin

### Phase huileuse :

- Montanov 68 (Société Seppic) 4,5 %
- Sel disodique de l'acide N-Stéaroyl L-glutamique (Acylglutamate HS21 de la société AJINOMOTO) 0,5 %
- Huile de jojoba (P.M. de l'ordre de 900) 9 %
- Huile d'avocat (P.M. de l'ordre de 900) 9 %
- Huile de silicone volatile (P.M. 106) 6 %

### Phase aqueuse :

- Glycérine 5 %
- Dipropylène glycol 10 %
- Eau 56 %

On obtient une nanoémulsion transparente gélifiée dont la taille des globules est de 46 nm et la turbidité de 240 NTU.

## Revendications

1. Nanoémulsion comportant une phase huileuse dispersée dans une phase aqueuse et ayant des globules d'huile dont la taille moyenne en nombre est inférieure à 100 nm, **caractérisée en ce qu'**elle contient un tensioactif solide à une température égale à 45°C, choisi parmi les esters d'acide gras et de sucre et les éthers d'alcool gras et de sucre, et au moins une huile ayant un poids moléculaire supérieur à 400, et **en ce que** le rapport en poids de la quantité de phase huileuse sur la quantité de tensioactif va de 2 à 10.

2. Nanoémulsion selon la revendication 1, **caractérisée en ce qu'**elle a une turbidité allant de 60 à 600 NTU.

3. Nanoémulsion selon la revendication 1 ou 2, **caractérisée en ce que** la quantité de tensioactif va de 0,2 à 15 % en poids et de préférence de 1 à 8 % en poids par rapport au poids total de la nanoémulsion.

4. Nanoémulsion selon l'une quelconque des revendications précédentes, **caractérisée en ce que** le rapport en poids de la quantité de phase huileuse sur la quantité de tensioactif va de 3 à 6.

5. Nanoémulsion selon l'une quelconque des revendications précédentes, **caractérisée en ce que** les globules d'huile ont une taille moyenne allant de 20 à 75 nm.

6. Nanoémulsion selon l'une quelconque des revendications précédentes, **caractérisée en ce que** le tensioactif est choisi dans le groupe comprenant les esters ou les mélanges d'esters d'acide gras en C₈-C₂₂ et de sucrose, de maltose, de glucose ou de fructose, les esters ou les mélanges d'esters d'acide gras en C₁₄-C₂₂ et de méthylglucose, les éthers ou mélanges d'éthers d'alcool gras en C₈-C₂₂ et de glucose, de maltose, de sucrose ou de fructose et les éthers ou mélanges d'éthers d'alcool gras en C₁₄-C₂₂ et de méthylglucose.

7. Nanoémulsion selon l'une quelconque des revendications précédentes, **caractérisée en ce que** le tensioactif est choisi dans le groupe comprenant le monostéarate de sucrose, le distéarate de sucrose, le tristéarate de sucrose et leurs mélanges, le distéarate de méthyl glucose et de polyglycérol-3, les alkylpolyglucosides.

8. Nanoémulsion selon l'une quelconque des revendications précédentes, **caractérisée en ce que** l'huile de poids moléculaire supérieur à 400 est choisie parmi les huiles d'origine animale ou végétale, les huiles minérales, les huiles de synthèse et les huiles de silicone, et leurs mélanges.

9. Nanoémulsion selon l'une quelconque des revendications précédentes, **caractérisée en ce que** la phase huileuse contient en outre au moins une huile ayant un poids moléculaire inférieur à 400.

10. Nanoémulsion selon l'une quelconque des revendications précédentes, **caractérisée en ce que** la phase huileuse contient au moins 40 % en poids d'huile(s) ayant un poids moléculaire supérieur à 400, par rapport au poids total de la phase huileuse.

11. Nanoémulsion selon l'une quelconque des revendications précédentes, **caractérisée en ce que** la quantité de phase huileuse va de 2 à 40 % en poids par rapport au poids total de la nanoémulsion.

12. Nanoémulsion selon l'une quelconque des revendications précédentes, **caractérisée en ce qu'**elle contient en outre au moins un lipide amphiphile ionique choisi dans le groupe formé par les lipides amphiphiles anioniques, les lipides amphiphiles cationiques, les dérivés alkylsulfoniques.

13. Nanoémulsion selon la revendication 12, **caractérisée en ce que** les lipides amphiphiles ioniques sont choisis dans le groupe formé par :
- les sels alcalins du dicetyl- et du dimyristylphosphate ;
- les sels alcalins du cholestérol sulfate ;
- les sels alcalins du cholestérol phosphate ;
- les sels de lipoaminoacides ;
- les sels de sodium de l'acide phosphatidique ;
- les phospholipides ;
- les dérivés alkylsulfoniques de formule (I) : 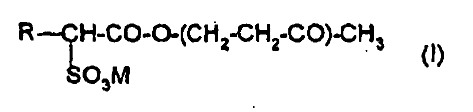 dans laquelle R représente des radicaux alkyle en C₁₆-C_{22,} pris en mélange ou séparément et M est un métal alcalin ;
- les sels d'ammonium quaternaire, les amines grasses et leurs sels ;
et leurs mélanges.

14. Nanoémulsion selon la revendication 12 ou 13, **caractérisée en ce que** la quantité de lipide(s) amphiphile(s) ionique(s) va de 0,01 à 5 % en poids par rapport au poids total de la nanoémulsion.

15. Nanoémulsion selon l'une quelconque des revendications précédentes, **caractérisée en ce qu'**elle contient un additif permettant d'améliorer la transparence, choisi parmi les alcools inférieurs comportant de 1 à 8 atomes de carbone, les glycols, les sucres et leurs mélanges.

16. Nanoémulsion selon la revendication 15, **caractérisée en ce que** l'additif est présent en une concentration allant de 5 à 20 % en poids par rapport au poids total de la nanoémulsion.

17. Nanoémulsion selon l'une quelconque des revendications précédentes,
**caractérisée en ce qu'**elle contient un actif cosmétique, dermatologique ou ophtalmologique.

18. Composition à usage topique, **caractérisée en ce qu'**elle est constituée d'une nanoémulsion selon l'une quelconque des revendications 1 à 17.

19. Support ophtalmique, **caractérisé en ce qu'**il est constitué d'une nanoémulsion selon l'une quelconque des revendications 1 à 17.

20. Utilisation non-thérapeutique de la nanoémulsion selon l'une quelconque des revendications 1 à 17, pour le soin, le traitement et/ou le maquillage de la peau, du visage et/ou du cuir chevelu.

21. Utilisation non-thérapeutique de la nanoémulsion selon l'une quelconque des revendications 1 à 17, pour le soin et/ou le traitement des cheveux.

22. Procédé non-thérapeutique de soin et/ou d'hydratation de la peau, des muqueuses et/ou du cuir chevelu, **caractérisé en ce que** qu'on applique sur la peau, les muqueuses et/ou sur le cuir chevelu une nanoémulsion selon l'une quelconque des revendications 1 à 17.

23. Utilisation de la nanoémulsion selon l'une quelconque des revendications 1 à 17, pour la fabrication d'une composition dermatologique destinée au traitement de la peau sèche.

24. Utilisation de la nanoémulsion selon l'une quelconque des revendications 1 à 17, pour la fabrication d'une composition ophtalmologique.

25. Procédé de préparation d'une nanoémulsion selon l'une quelconque des revendications 1 à 17, consistant à mélanger la phase aqueuse et la phase huileuse sous agitation vive, à une température ambiante allant de 10 à 80°C, puis à effectuer une homogénéisation à une pression allant de 6.10⁷ Pa à 18.10⁷ Pa.

26. Procédé selon la revendication précédente, **caractérisé en ce que** le cisaillement va de 2.10⁶ s⁻¹ à 5.10⁸ s⁻¹.

## Claims

1. Nanoemulsion comprising an oily phase dispersed in an aqueous phase and having oil globules with a number-average size of less than 100 nm, **characterized in that** it comprises a surfactant which is solid at a temperature equal to 45°C, which surfactant is chosen from esters of a fatty acid and of a sugar and ethers of a fatty alcohol and of a sugar, and at least one oil having a molecular weight of greater than 400 and **in that** the ratio by weight of the amount of oily phase to the amount of surfactant ranges from 2 to 10.

2. Nanoemulsion according to Claim 1, **characterized in that** it has a turbidity ranging from 60 to 600 NTU.

3. Nanoemulsion according to Claim 1 or 2, **characterized in that** the amount of surfactant ranges from 0.2 to 15% by weight and preferably from 1 to 8% by weight with respect to the total weight of the nanoemulsion.

4. Nanoemulsion according to any one of the preceding claims, **characterized in that** the ratio by weight of the amount of oily phase to the amount of surfactant ranges from 3 to 6.

5. Nanoemulsion according to any one of the preceding claims, **characterized in that** the oil globules have an average size ranging from 20 to 75 nm.

6. Nanoemulsion according to any one of the preceding claims, **characterized in that** the surfactant is chosen from the group consisting of esters or mixtures of esters of a C₈-C₂₂ fatty acid and of sucrose, maltose, glucose or fructose, esters or mixtures of esters of a C₁₄-C₂₂ fatty acid and of methylglucose, ethers or mixtures of ethers of a C₈-C₂₂ fatty alcohol and of glucose, maltose, sucrose or fructose, and ethers or mixtures of ethers of a C₁₄-C₂₂ fatty alcohol and of methylglucose.

7. Nanoemulsion according to any one of the preceding claims, **characterized in that** the surfactant is chosen from the group comprising sucrose monostearate, sucrose distearate, sucrose tristearate and their mixtures, the distearate of methylglucose and of polyglycerol-3, and alkyl polyglucosides.

8. Nanoemulsion according to any one of the preceding claims, **characterized in that** the oil with a molecular weight of greater than 400 is chosen from oils of animal or vegetable origin, mineral oils, synthetic oils and silicone oils, and their mixtures.

9. Nanoemulsion according to any one of the preceding claims, **characterized in that** the oily phase additionally comprises at least one oil having a molecular weight of less than 400.

10. Nanoemulsion according to any one of the preceding claims, **characterized in that** the oily phase comprises at least 40% by weight of oil(s) having a molecular weight of greater than 400 with respect to the total weight of the oily phase.

11. Nanoemulsion according to any one of the preceding claims, **characterized in that** the amount of oily phase ranges from 2 to 40% by weight with respect to the total weight of the nanoemulsion.

12. Nanoemulsion according to any one of the preceding claims, **characterized in that** it additionally comprises at least one ionic amphiphilic lipid chosen from the group formed by anionic amphiphilic lipids, cationic amphiphilic lipids and alkylsulphonic derivatives.

13. Nanoemulsion according to Claim 12, **characterized in that** the ionic amphiphilic lipids are chosen from the group formed by:
- the alkaline salts of dicetyl and dimyristyl phosphate;
- the alkaline salts of cholesterol sulphate;
- the alkaline salts of cholesterol phosphate;
- the salts of lipoamino acids;
- the sodium salts of phosphatidic acid;
- phospholipids;
- the alkylsulphonic derivatives of formula (I): 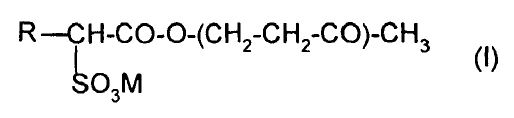 in which R represents C₁₆-C₂₂ alkyl radicals, taken as a mixture or separately, and M is an alkali metal;
- quaternary ammonium salts, fatty amines and their salts;
and their mixtures.

14. Nanoemulsion according to Claim 12 or 13, **characterized in that** the amount of ionic amphiphilic lipid(s) ranges from 0.01 to 5% by weight with respect to the total weight of the nanoemulsion.

15. Nanoemulsion according to any one of the preceding claims, **characterized in that** it comprises an additive which makes it possible to improve the transparency chosen from lower alcohols comprising from 1 to 8 carbon atoms, glycols, sugars and their mixtures.

16. Nanoemulsion according to Claim 15, **characterized in that** the additive is present in a concentration ranging from 5 to 20% by weight with respect to the total weight of the nanoemulsion.

17. Nanoemulsion according to any one of the preceding claims, **characterized in that** it comprises a cosmetic, dermatological or ophthalmological active principle.

18. Composition for topical use, **characterized in that** it is composed of a nanoemulsion according to any one of Claims 1 to 17.

19. Ophthalmic support, **characterized in that** it is composed of a nanoemulsion according to any one of Claims 1 to 17.

20. Non-therapeutic use of the nanoemulsion according to any one of Claims 1 to 17 for caring for, treating and/or making up the skin, face and/or scalp.

21. Non-therapeutic use of the nanoemulsion according to any one of Claims 1 to 17 for caring for and/or treating the hair.

22. Non-therapeutic process for caring for and/or moisturizing the skin, mucous membranes and/or scalp, **characterized in that** a nanoemulsion according to any one of Claims 1 to 17 is applied to the skin, the mucous membranes and/or to the scalp.

23. Use of the nanoemulsion according to any one of Claims 1 to 17 in the manufacture of a dermatological composition intended for the treatment of dry skin.

24. Use of the nanoemulsion according to any one of Claims 1 to 17 in the manufacture of an ophthalmological composition.

25. Process for the preparation of a nanoemulsion according to any one of Claims 1 to 17 which comprises the mixing of the aqueous phase and the oily phase with vigorous stirring at an ambient temperature ranging from 10 to 80°C and then a homogenization of the mixture at a pressure ranging from 6 × 10⁷ Pa to 18 × 10⁷ Pa.

26. Process according to the preceding claim, **characterized in that** the shearing ranges from 2 × 10⁶ s⁻¹ to 5 × 10⁸ s⁻¹.

## Patentansprüche

1. Nanoemulsion, die eine in einer wässrigen Phase dispergierte Ölphase mit Ölkügelchen aufweist, wobei das Zahlenmittel der Größe der Ölkügelchen unter 100 nm liegt, **dadurch gekennzeichnet, dass** sie einen grenzflächenaktiven Stoff, der bei einer Temperatur von 45 °C fest ist und der unter den Zuckerfettsäureestern und den Zuckerfettalkoholethern ausgewählt ist, und mindestens ein Öl mit einer Molmasse über 400 enthält und **dadurch**, dass das Gewichtsverhältnis der Menge der Ölphase und der Menge des grenzflächenaktiven Stoffes im Bereich von 2 bis 10 liegt.

2. Nanoemulsion nach Anspruch 1, **dadurch gekennzeichnet, dass** sie eine Trübung von 60 bis 600 NTU aufweist.

3. Nanoemulsion nach Anspruch 1 oder 2, **dadurch gekennzeichnet, dass** die Menge des grenzflächenaktiven Stoffes im Bereich von 0,2 bis 15 Gew.-% und vorzugsweise 1 bis 8 Gew.-%, bezogen auf das Gesamtgewicht der Nanoemulsion, liegt.

4. Nanoemulsion nach einem der vorhergehenden Ansprüche, **dadurch gekennzeichnet, dass** das Gewichtsverhältnis der Menge der Ölphase und der Menge des grenzflächenaktiven Stoffes im Bereich von 3 bis 6 liegt.

5. Nanoemulsion nach einem der vorhergehenden Ansprüche, **dadurch gekennzeichnet, dass** die Ölkügelchen eine mittlere Größe von 20 bis 75 nm aufweisen.

6. Nanoemulsion nach einem der vorhergehenden Ansprüche, **dadurch gekennzeichnet, dass** der grenzflächenaktive Stoff unter den Estern oder Gemischen von Estern einer Fettsäure mit 8 bis 22 Kohlenstoffatomen und Saccharose, Maltose, Glucose oder Fructose und den Estern oder Gemischen von Estern einer Fettsäure mit 14 bis 22 Kohlenstoffatomen und Methylglucose, den Ethern oder Gemischen von Ethern eines Fettalkohols mit 8 bis 22 Kohlenstoffatomen und Glucose, Maltose, Saccharose oder Fructose und den Ethern oder Gemischen von Ethern eines Fettalkohols mit 14 bis 22 Kohlenstoffatomen und Methylglucose ausgewählt ist.

7. Nanoemulsion nach einem der vorhergehenden Ansprüche, **dadurch gekennzeichnet, dass** der grenzflächenaktive Stoff unter Saccharosemonostearat, Saccharosedistearat, Saccharosetristearat und deren Gemischen, dem Distearat von Methylglucose und Polyglycerol-3 und den Alkylpolyglucosiden ausgewählt ist.

8. Nanoemulsion nach einem der vorhergehenden Ansprüche, **dadurch gekennzeichnet, dass** das Öl mit einer Molmasse über 400 unter den Ölen tierischer oder pflanzlicher Herkunft, Mineralölen, synthetischen Ölen und Siliconölen und deren Gemischen ausgewählt ist.

9. Nanoemulsion nach einem der vorhergehenden Ansprüche, **dadurch gekennzeichnet, dass** die Ölphase auch mindestens ein Öl mit einer Molmasse unter 400 enthält.

10. Nanoemulsion nach einem der vorhergehenden Ansprüche, **dadurch gekennzeichnet, dass** die Ölphase mindestens 40 Gew.-% Öl(e) mit einer Molmasse über 400, bezogen auf das Gesamtgewicht der Ölphase, enthält.

11. Nanoemulsion nach einem der vorhergehenden Ansprüche, **dadurch gekennzeichnet, dass** der Mengenanteil der Ölphase im Bereich von 2 bis 40 Gew.-%, bezogen auf das Gesamtgewicht der Nanoemulsion, liegt.

12. Nanoemulsion nach einem der vorhergehenden Ansprüche, **dadurch gekennzeichnet, dass** sie ferner mindestens ein ionisches amphiphiles Lipid enthält, das unter den anionischen amphiphilen Lipiden, den kationischen amphiphilen Lipiden und den Alkylsulfonsäurederivaten ausgewählt ist.

13. Nanoemulsion nach Anspruch 12, **dadurch gekennzeichnet, dass** die ionischen amphiphilen Lipide ausgewählt sind unter:
- den Alkalisalzen von Dicetyl- und Dimyristylphosphat;
- den Alkalisalzen von Cholesterinsulfat;
- den Alkalisalzen von Cholesterinphosphat;
- den Salzen von Lipoaminosäuren;
- den Natriumsalzen von Phosphatidsäure;
- den Phospholipiden;
- den Alkylsulfonsäurederivaten der Formel (I): 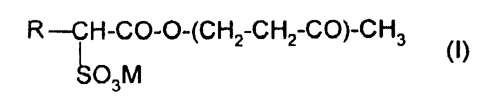 worin R als Gemisch oder einzeln Alkylgruppen mit 16 bis 22 Kohlenstoffatomen bedeutet und M ein Alkalimetall ist,
- den quartären Ammoniumsalzen, den Fettaminen und deren Salzen; und
- ihren Gemischen;

14. Nanoemulsion nach Anspruch 12 oder 13, **dadurch gekennzeichnet, dass** die Menge des oder der ionischen amphiphilen Lipide(s) im Bereich von 0,01 bis 5 Gew.-%, bezogen auf das Gesamtgewicht der Nanoemulsion, liegt.

15. Nanoemulsion nach einem der vorhergehenden Ansprüche, **dadurch gekennzeichnet, dass** sie einen Zusatzstoff enthält, der die Transparenz verbessern kann und der unter den niederen Alkoholen mit 1 bis 8 Kohlenstoffatomen, Glycolen, Zuckern und deren Gemischen ausgewählt ist.

16. Nanoemulsion nach dem Anspruch 15, **dadurch gekennzeichnet, dass** der Zusatzstoff in einer Konzentration von 5 bis 20 Gew.-%, bezogen auf das Gesamtgewicht der Nanoemulsion, vorliegt.

17. Nanoemulsion nach einem der vorhergehenden Ansprüche, **dadurch gekennzeichnet, dass** sie einen kosmetischen, dermatologischen oder ophthalmologischen Wirkstoff enthält.

18. Zusammensetzung zur topischen Anwendung, **dadurch gekennzeichnet, dass** sie von einer Nanoemulsion nach einem der Ansprüche 1 bis 17 gebildet wird.

19. Ophthalmischer Träger, **dadurch gekennzeichnet, dass** er von einer Nanoemulsion nach einem der Ansprüche 1 bis 17 gebildet wird.

20. Nicht therapeutische Verwendung der Nanoemulsion nach einem der Ansprüche 1 bis 17 zur Pflege, zur Behandlung und/oder zum Schminken der Haut, des Gesichts und/oder der Kopfhaut.

21. Nicht therapeutische Verwendung der Nanoemulsion nach einem der Ansprüche 1 bis 17 zur Pflege und/oder Behandlung der Haare.

22. Nicht therapeutisches Verfahren zur Pflege und/oder Hydratisierung der Haut, der Schleimhäute und/oder der Kopfhaut, **dadurch gekennzeichnet, dass** auf die Haut, die Schleimhäute und/oder die Kopfhaut eine Nanoemulsion nach einem der Ansprüche 1 bis 17 aufgetragen wird.

23. Verwendung der Nanoemulsion nach einem der Ansprüche 1 bis 17 zur Herstellung einer dermatologischen Zusammensetzung, die zur Behandlung von trockener Haut vorgesehen ist.

24. Verwendung der Nanoemulsion nach einem der Ansprüche 1 bis 17 zur Herstellung einer ophthalmologischen Zusammensetzung.

25. Verfahren zur Herstellung einer Nanoemulsion nach einem der Ansprüche 1 bis 17, das darin besteht, die wässrige Phase und die Ölphase unter kräftigem Rühren bei einer Umgebungstemperatur von 10 bis 80 °C zu vermischen und dann eine Homogenisierung bei einem Druck von 6 · 10⁷ bis 18 · 10⁷ Pa durchzuführen.

26. Verfahren nach dem vorhergehenden Anspruch, **dadurch gekennzeichnet, dass** die Scherbeanspruchung im Bereich von 2 · 10⁶ bis 5 · 10⁸ s⁻¹ liegt.
